(19) Europäisches Patentamt
European Patent Office
Office européen des brevets

(11) Veröffentlichungsnummer : **0 155 474**
**B1**

(12) # EUROPÄISCHE PATENTSCHRIFT

(45) Veröffentlichungstag der Patentschrift : **11.01.89**

(51) Int. Cl.[4] : **C 07 C149/36**, C 09 K 15/14, C 08 K 5/37

(21) Anmeldenummer : **85101004.1**

(22) Anmeldetag : **31.01.85**

(54) **Bis-,Tris-, Tetrakis-und Pentakis-(substituiertes hydroxy-phenylthio)-alkane und-cycloalkane und sie enthaltende Stoffzusammensetzungen.**

(30) Priorität : **03.02.84 US 576978**

(43) Veröffentlichungstag der Anmeldung : **25.09.85 Patentblatt 85/39**

(45) Bekanntmachung des Hinweises auf die Patenterteilung : **11.01.89 Patentblatt 89/02**

(84) Benannte Vertragsstaaten : **DE FR GB IT**

(56) Entgegenhaltungen :
**EP-A- 0 119 160**
**DE-B- 1 768 334**
**US-A- 3 489 804**

(73) Patentinhaber : **CIBA-GEIGY AG**
**Klybeckstrasse 141**
**CH-4002 Basel (CH)**

(72) Erfinder : **Spivack, John D.**
**1 Blue Jay Street**
**Spring Valley New York 10977 (US)**
Erfinder : **Pastor, Stephen D.**
**1080 Warburton Avenue**
**Yonkers New York 10701 (US)**

(74) Vertreter : **Zumstein, Fritz, Dr. et al**
**Bräuhausstrasse 4**
**D-8000 München 2 (DE)**

**EP 0 155 474 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Alkane oder Cycloalkane, die zwei-, drei-, vier- oder fünfmal durch eine substituierte Hydroxyphenylthiogruppe substituiert sind und ihre Verwendung als Stabilisatoren für organisches Material.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischen Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Ueberraschenderweise ist jetzt gefunden worden, dass die durch zwei Hydroxyphenylthiogruppen und eine oder zwei weiteren Thiogruppen substituierten Alkane oder Cycloalkane dieser Erfindung eine ungewöhnliche Kombination wünschenswerter Eigenschaften besitzen, wodurch diese Stoffe zu besonders effektiven Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Eine Reihe verwandter US-Patente der Nummern 3576883, 3786100, 3897500 und 3956359 beschreiben ketosubstituierte Alkylidendithiobisphenole, die durch das Vorhandensein von Bis-(3,5-di-tert.-butyl-4-hydroxyphenyl)-gruppen und verschiedener Alkylidenbrücken charakterisiert sind. In jedem Fall muss ein Methylrest am Alkyliden brückenglied der genannten Verbindungen gebunden sein. Die Verbindungen der obengenannten Patente sind als zur Senkung des Cholesterins im Blut von Warmblütlern geeignete Substanzen beschrieben. Kein Hinweis ist zu finden für deren Verwendung als Stabilisatoren. M.B. Neuworth et al., J. Medicinal Chemistry, 13, 722 (1970) beschreibt dieselben in den 4 oben erwähnten US-Patenten offenbarten Verbindungen sowie einige ähnliche Alkylidendithiophenole mit verschiedenen Substituenten am Phenylring und am Alkylidenbrückenglied. Auch in diesem Fall wird einzig und allein auf die cholesterinsenkende Wirkung dieser Substanzen hingewiesen. Die erfindungsgemässen Verbindungen unterscheiden sich strukturell ganz deutlich von den zweifach mit einer substituierten Hydroxyphenylthiogruppe besetzten Verbindungen der genannten Publikation.

Drei japanische Offenlegungsschriften, JP Patent Kokai 79/163536, 80/9041 und 80/17316 (entsprechend CA, 93, 71280 × (1980) ; CA, 93, 94980q (1980) und CA, 93, 149970u (1980)) beschreiben, 2,2-Bis-(3,5-di-tert.-butyl-4-hydroxyphenylthio)-propan ebenfalls als Cholesterinsenkendes Mittel.

US-Patent 3704327 beschreibt Bis-(3-methyl-5-tert.-butyl-4-hydroxyphenylthio) methan und 1,1-Bis-(3-methyl-5-tert.-butyl-4-hydroxy-phenylthio) ethan als Antioxidans für Kautschuk. Die erfindungsgemässen Verbindungen unterscheiden sich strukturell von diesen vorbekannten Verbindungen dadurch, dass sie eine zusätzliche Thioethergruppe enthalten.

Die vorliegende Erfindung betrifft Verbindungen der Formel I

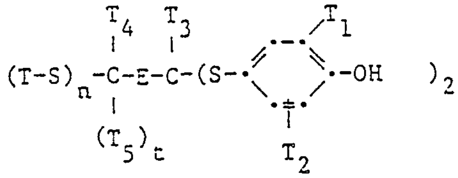

(I)

worin

$T_1$ und $T_2$ unabhängig voneinander $C_1$-$C_{12}$-Alkyl, $C_5$-$C_6$-Cycloalkyl, unsubstituiertes oder durch $C_1$-$C_{12}$-Alkyl substituiertes Phenyl, unsubstituiertes oder durch $C_1$-$C_{12}$-Alkyl-substituiertes $C_7$-$C_9$-Aralkyl bedeuten und $T_2$ zusätzlich Wasserstoff sein kann,

T $C_1$-$C_{24}$-Alkyl, unsubstituiertes oder durch $C_1$-$C_{12}$-Alkyl substituiertes Phenyl oder eine Gruppe

(Siehe Formel Seite 3 f.)

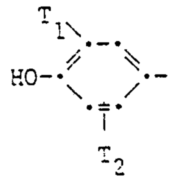 ist,

E eine direkte Bindung, $C_1$-$C_{11}$-Alkylen, $C_5$-$C_6$-Cycloalkylen, Phenylen oder die Gruppe

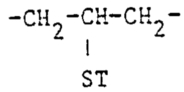 bedeutet,

$T_3$ und $T_4$ unabhängig voneinander Wasserstoff, $C_1$-$C_{30}$-Alkyl, welches unsubstituiert oder durch unsubstituiertes oder durch $C_1$-$C_{12}$-Alkyl substituiertes Phenyl substituiert sein kann, oder $C_5$-$C_6$-Cycloalkyl sind oder, wenn E Methylen bedeutet, $T_3$ und $T_4$ zusammen geradkettiges oder verzweigtes $C_3$-$C_7$-Alkylen bilden,

$T_5$ Wasserstoff, $C_1$-$C_4$-Alkyl oder Phenyl ist,

n die Zahlen 1 oder 2 und

t Null oder 1 sind, wobei t = 2—n sein muss.

Bevorzugt sind diejenigen erfindungsgemässen Verbindungen, worin $T_2$ in ortho-Stellung zur Hydroxygruppe steht.

Bedeuten die Substituenten T bis $T_5$ und/oder E jeweils Alkyl, Cycloalkyl, Aralkyl, Alkylen, Cycloalkylen und/oder Phenylen so handelt es sich beispielsweise um folgende Reste :

$C_1$-$C_{30}$-Alkyl ist z. B. Methyl, Ethyl, Isopropyl, n-Butyl, sec-Butyl, tert.-Butyl, Isoamyl, tert.-Amyl, n-Hexyl, 2-Ethylhexyl, n-Octyl, 1,1,3,3-Tetramethylbutyl, n-Dodecyl, tert.-Dodecyl, n-Octadecyl, Eicosyl oder Triaconyl.

$C_2$-$C_6$-Cycloalkyl bedeutet z. B. Cyclopentyl oder Cyclohexyl.

$C_7$-$C_9$-Aralkyl ist beispielsweise Benzyl, α-Methylbenzyl oder α,α-Dimethylbenzyl.

$C_1$-$C_{11}$-Alkylen ist. z. B. Methylen, Ethylen, Propylen, Trimethylen, 2,2-Dimethylpropan-1,3-diyl, Tetramethylen, Pentamethylen, Octamethylen oder Undecamethylen.

$C_5$-$C_6$-Cycloalkylen bedeutet z. B. Cyclopentylen oder 1,4-Cyclohexylen.

Phenylen ist o-Phenylen, m-Phenylen oder p-Phenylen.

$T_1$ und $T_2$ sind vorzugsweise unabhängig voneinander geradkettiges oder verzweigtes $C_1$-$C_8$-Alkyl, wie z. B. Methyl, Isopropyl, n-Butyl, sec-Butyl, tert.-Butyl, tert.-Amyl oder tert.-Octyl. Besonders bevorzugt sind sie Methyl, tert.-Butyl, tert.-Amyl und 1,1,3,3-Tetramethylbutyl.

Besonders bevorzugt sind $T_1$ Methyl oder tert.-Butyl und $T_2$ tert.-Butyl.

Ganz besonders bevorzugt sind $T_1$ und $T_2$ in ortho-Stellung zur Hydroxygruppe und beide tert.-Butyl.

T ist vorzugsweise $C_1$-$C_{18}$-Alkyl, insbesondere $C_7$-$C_{18}$-Alkyl oder die Gruppe

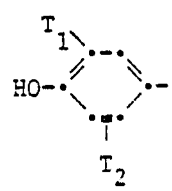

E ist bevorzugt eine direkte Bindung, $C_1$-$C_6$-Alkylen oder die Gruppe

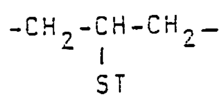

$T_3$ und $T_4$ sind vorzugsweise unabhängig voneinander Wasserstoff oder $C_1$-$C_4$-Alkyl.

Bilden $T_3$ und $T_4$ zusammen $C_3$-$C_7$-Alkylen, so handelt es sich bevorzugt um Trimethylen oder 2,2-Dimethylpropan-1,3-diyl.

$T_5$ ist bevorzugt Wasserstoff oder Methyl.

Die Verbindungen der vorliegenden Erfindung können z. B. durch Umsetzung eines geeigneten 4-Mercaptophenols mit einer entsprechenden Bis-carbonylverbindung in Gegenwart einer starken Säure, wie zum Beispiel Salzsäure, als Katalysator hergestellt werden. Die Umsetzung wird vorzugsweise in einem inerten organischen Lösungsmittel, wie beispielsweise Methanol, Heptan, Benzol oder Toluol durchgeführt. Die Reaktionstemperatur kann von 0° bis 100 °C variieren. Die Ausgangssubstanzen sind bekannt und im allgemeinen im Handel erhältlich. Sollten einige noch neu sein, so sind sie Analogie zu allgemein bekannten Methoden herstellbar.

Wenn erfindungsgemässe Verbindungen der Formel I, worin n = 1 ist, aus α,β-ungesättigten Carbonylverbindungen hergestellt werden, so wird zweckmäßig vorerst das geeignete Mercaptan oder 4-Mercaptophenol an die activierte Doppelbindung in Gegenwart eines tertiären Amins, wie z. B. Triethylamin, als Katalysator addiert. Danach folgt die Reaktion des geeigneten 4-Mercaptophenols mit der Carbonylgruppe in Gegenwart einer starken Säure, wie beispielsweise Bor-trifluoridetherat als Katalysator.

Die erfindungsgemässen Verbindungen lassen sich als effektive Stabilisatoren für Kunststoffe, Polymere oder Harze einsetzen. Sie lassen sich auch als Stabilisatoren in Mineralölen oder synthetischen Oelen, wie Schmierölen, Umlaufölen und dergleichen verwenden.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer zu verstehen ist.

Vorzugsweise verwendet man die Verbindungen der Formel I als Stabilisatoren für homopolymere oder copolymere Polyolefine sowie für Polystyrol, einschliesslich schlagfestem Polystyrol, ABS-Harz, SBR, Polyisopren, natürlicher Kautschuk, Polyester, einschliesslich Polyethylenterephthalat und Polybutylenterephthalat und Copolymere sowie für Schmiermittel, wie beispielsweise die aus Mineralölen gewonnenen.

Polymere, die mit den erfindungsgemässen Verbindungen stabilisiert werden können, sind beispielsweise

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere. Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen

Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den enstsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyester-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäure-estern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten. z. B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z. B.) Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die erfindungsgemässen Stabilisatoren werden den Kunststoffen z. B. in Mengen von 0,01 bis 5 Gew.-% bezogen auf das stabilisierte Material zugemischt. Je nach Verwendung und Substrat wird die Stabilisatormenge variiert. Bevorzugt verwendet man 0,05 bis 2 Gew.-% des Stabilisators, besonders bevorzugt 0,1 bis 1 Gew.-%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine Suspension des Stabilisators kann mit einer Lösung, Suspension oder Emulsion des Polymeren vermischt werden.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index Verbesserer, Stockpunkterniedriger, Dispergiermittel/Tenside und Verschleiss-schutzadditive. Als weitere Additive, mit denen zusammen die erfindungsgemäss verwendeten Stabilisatoren eingesetzt werden können, sind beispielsweise zu nennen :

1. Antioxidantien

1.1. Alkylierte Monophenole

2,6-Di-tert. butyl-4-methylphenol
2-Tert. butyl-4,6-dimethylphenol

2,6-Di-tert. butyl-4-ethylphenol
2,6-Di-tert. butyl-4-n-butylphenol
2,6-Di-tert. butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert. butyl-4-methoxymethylphenol

1.2. Alkylierte Hydrochinone

2,6-Di-tert. butyl-4-methoxyphenol
2,5-Di-tert. butyl-hydrochinon
2,5-Di-tert. amyl-hydrochinon
2,6-Diphenyl-4-octadecyloxyphenol

1.3. Hydroxylierte Thiodiphenylether

2,2'-Thio-bis-(6-tert. butyl-4-methylphenol)
2,2'-Thio-bis-(4-octylphenol)
4,4'-Thio-bis-(6-tert. butyl-3-methylphenol)
4,4'-Thio-bis-(6-tert. butyl-2-methylphenol)

1.4. Alkyliden-Bisphenole

2,2'-Methylen-bis-(6-tert. butyl-4-methylphenol)
2,2'-Methylen-bis-(6-tert. butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis (4,6-di-tert. butylphenol)
2,2'-Ethyliden-bis-(4,6-di-tert. butylphenol)
2,2'-Ethyliden-bis-(6-tert. butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert. butyl-2-methylphenol)
1,1-Bis-(5-tert. butyl-4-hydroxy-2-methylphenyl)-butan
2,6-Di-(3-tert. butyl-5-methyl-2-hydroxybenzyl)-4-methylphenol
1,1,3-Tris-(5-tert. butyl-4-hydroxy-2-methylphenyl)-butan
1,1-Bis-(5-tert. butyl-4-hydroxy-2-methylphenyl)-3-n-dodecylmercaptobutan
Ethylenglycol-bis-[3,3-bis-(3'-tert. butyl-4'-hydroxyphenyl)-butyrat]
Di-(3-tert. butyl-4-hydroxy-5-methylphenyl)-dicyclopentadien
Di-[2-(3'-tert. butyl-2'-hydroxy-5'-methyl-benzyl)-6-tert. butyl-4-methyl-phenyl]-terephthalat.

1.5 Benzylverbindungen

1,3,5-Tri-(3,5-di-tert. butyl-4-hydroxybenzyl)-2,4,6-trimethylbenzol
Di-(3,5-di-tert. butyl-4-hydroxybenzyl)-sulfid
3,5-di-tert. butyl-4-hydroxybenzyl-mercaptoessigsäure-isooctylester
Bis-(4-tert. butyl-3-hydroxy-2,6-dimethylbenzyl) dithiol-terephthalat
1,3,5-Tris-(3,5-di-tert. butyl-4-hydroxybenzyl)-isocyanurat
1,3,5-Tris-(4-tert. butyl-3-hydroxy-2,6-dimethylbenzyl)-isocyanurat
3,5-Di-tert. butyl-4-hydroxybenzyl-phosphonsäure-dioctadecylester
3,5-Di-tert. butyl-4-hydroxybenzyl-phosphonsäure-monoethylester, Calcium-salz.

1.6. Acylaminophenole

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert. butyl-4-hydroxyphenyl)-carbaminsäureoctylester.

1.7. Ester der β-(3,5-Di-tert. butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der β-(5-tert. butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert. butyl-4-hydroxyphenyl)-propionsäure, wie z. B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z. B. das 5'-Methyl-,3',5'-Di-tert. butyl-, 5'-Tert. butyl-, 5'-(1,1,3,3-Tetramethylbutyl)-, 5-Chlor-3',5'-di-tert.butyl-, 5-Chlor-3'-tert. butyl-5'-methyl-, 3'-sec. Butyl-5'-tert. butyl, 4'-Octoxy-, 3',5'-Di-tert. amyl-, 3',5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-4,2',4'-Trihydroxy-, 2'-Hydroxy-4,4'-dimethoxy-Derivat.

2.3. Ester von gegebenenfalls substituierten Benzoesäuren, wie z. B. 4-Tert. butyl-phenylsalicylat, Phenylsalicylat, Octylphenyl-salicylat, Dibenzoylresorcin, Bis-(4-tert. butylbenzoyl)-resorcin, Benzoylresorcin, 3,5-Di-tert. butyl-4-hydroxybenzoesäure-2,4-di-tert. butylphenylester, 3,5-Di-tert. butyl-4-hydroxybenzoesäurehexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin. Triethanolamin oder N-Cyclohexyl-diethanolamin, Nickeldibutyldithiocarbamat, Nickelsalze von 4-Hydroxy-3,5-di-tert. butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2,2,6,6-tetramethyl-piperidyl)-sebacat
Bis-(1,2,2,6,6-pentamethylpiperidyl)-sebacat
n-Butyl-3,5-di-tert. butyl-4-hydroxybenzyl-malonsäure-bis(1,2,2,6,6-pentamethylpiperidyl)-ester, Kondensationsprodukt aus 1-Hydroxy-ethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert. Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetracarbonsäure,
1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert. butyl-oxanilid, 2,2'-Di-dodecyloxy-5,5'-di-tert. butyl-oxanilid, 2-Ethoxy-2'-ethyl-oxanilid, N,N'-Bis-(3-dimethylaminopropyl)-oxalamid, 2-Ethoxy-5-tert. butyl-2'-ethyl-oxanilid und dessen Gemisch mit 2-Ethoxy-2'-ethyl-5,4'-di-tert. butyl-oxanilid, Gemische von ortho-und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert. butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit,Trilaurylphosphit, Trioctadecylphosphit, Distearyl-pentaerythritdi-phosphit,Tris-(2,4-di-tert. butylphenyl)-phosphit, Diisodecylpentaerythrit-diphosphit, Di-(2,4-di-tert. butylphenyl)-pentaerythritdiphosphit, Tristearyl-sorbit-triphosphit,Tetrakis-(2,4-di-tert. butylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert. Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Für den Einsatz zusammen mit den erfindungsgemässen Verbindungen bevorzugte Costabilisatoren sind Antioxidantien, Lichtschutzmittel, Metalldesaktivatoren, Phosphite, Phosphonite und Thiosynergisten (peroxidzerstörende Verbindungen).

Die folgenden Beispiele erläutern die Erfindung. Alle Mengenangaben entsprechen Gewichtsteilen soweit nicht anderweitig angegeben.

Beispiel 1 : 1,1,2,2-Tetrakis(3,5-di-tert.-butyl-4-hydroxyphenylthio)-ethan

Eine Lösung von 23,84 g 2,6-Di-tert.-butyl-4-mercaptophenol und 3,63 g 40 %igem wässrigem Glyoxal in 150 ml Methanol wird in einem Kolben unter Stickstoff mit wasserfreiem Chlorwasserstoff behandelt und die Temperatur dabei auf 60 °C ansteigen gelassen. Das Reaktionsprodukt wird abfiltriert und aus Acetonitril/Toluol umkristallisiert. Man erhält 21,65 g (89 % d. Th.) einer weissen kristallinen Substanz mit Smp. 221-223 °C.

Analyse für $C_{58}H_{86}O_4S_4$
Berechnet : C 71,4 H 8,9
Gefunden : C 71,5 H 9,0.

Beispiel 2 : 1,1,5,5-Tetrakis-(3,5-di-tert.-butyl-4-hydroxyphenylthio)-pentan

Eine Lösung von 23,84 g 2,6-Di-tert.-butyl-4-mercaptophenol und 5,01 g 50 %igem wässrigem Glutaraldehyd in 150 ml Methanol wird in einem Kolben unter Stickstoff mit wasserfreiem Chlorwasserstoff behandelt und die Temperatur dabei auf 60 °C ansteigen gelassen. Es wird über Nacht gerührt, das erhaltene Produkt abfiltriert, ein erstes Mal aus Acetonitril und dann aus Heptan umkristallisiert. Man erhält 16,73 g (66 % d. Th.) einer weissen kristallinen Substanz mit Smp. 148-153 °C.

Analyse für $C_{61}H_{92}O_4S_4$
Berechnet : C 72,0 H 9,1
Gefunden : C 72,1 H 8,9.

Beispiel 3 : 1,1,3-Tris-(3,5-di-tert.-butyl-4-hydroxyphenylthio)-butan

a) 3-(3,5-Di-tert.-butyl-4-hydroxyphenylthio)-n-butyraldehyd

3,12 g Crotonaldehyd (0,042 Mol) werden tropfenweise innerhalb von 15 Minuten bei 17-22 °C einer Lösung von 10,8 g 2,6-Di-tert.-butyl-4-mercaptophenol (0,044 Mol) und 0,5 g Triethylamin in 20 ml Toluol zugegeben und unter Stickstoff zuerst 30 Minuten bei Zimmertemperatur und dann zwei Stunden bei 50-55 °C reagieren gelassen. Die Analyse zeigt, dass die Reaktion zu diesem Zeitpunkt praktisch beendet ist. Das als leicht gelbe Flüssigkeit isolierte Produkt wird in praktisch quantitativer Ausbeute rein erhalten nach Abdampfen der flüchtigen Anteile bei 40-50 °C und 0,195 mbar.

b) Das unter (a) erhaltene Produkt (14 g, 0,042 Mol) und 20,4 g 2,6-Di-tert.-butyl-4-mercaptophenol (0,084 Mol) werden zusammen in 85 ml Toluol gelöst. Zu dieser Lösung werden unter Rühren 0,3 g Bortrifluorid-etherat zugegeben. Das Reaktionsgemisch wird drei Stunden bei 50-55 °C gerührt. Die Analyse zeigt danach, dass das Mercaptophenol vollständig aufgebraucht ist und, dass ein kleiner Teil des Reagens noch im Reaktionsgemisch vorhanden ist. Es werden deshalb noch 2,43 g Mercaptophenol zugegeben und das Reaktionsgemisch einige Stunden weitergerührt. Zwei weitere Portionen Mercaptophenol (3,64 und 0,5 g) werden noch zugefügt und wie oben erwähnt weiterreagieren gelassen, bis durch Analyse festgestellt wird, dass die Reaktion weitgehend beendet ist. Das Reaktionsgemisch wird danach mit 2 N Natriumhydroxid-Lösung und anschliessend mit Wasser bis zum pH 6-7 gewaschen und über wasserfreiem Natriumsulfat getrocknet. Das Produkt wird durch Abfiltrieren des Trocknungsmittels und Abdestillieren des Lösungsmittels im Vakuum isoliert. Durch Umkristallisieren aus n-Heptan erhält man eine weisse kristalline Substanz mit Smp. 125-126,5 °C.

Analyse für $C_{46}H_{70}O_3S_3$
Berechnet : C 72,01 H 9,2
Gefunden : C 71,08 H 8,9.

Beispiel 4 : 1,1-Bis-(3,5-di-tert.-butyl-4-hydroxyphenylthio)-3-(n-dodecylthio)-butan

a) 3-(n-Dodecylthio)-n-butyraldehyd

14,02 g Crotonaldehyd (0,2 Mol) werden innerhalb von 40 Minuten bei 60-65 °C einer Lösung von 41,2 g n-Dodecylmercaptan (0,2 Mol) und 0,66 g Triethylamin unter Rühren zugetropft. Man heizt und rührt weiter bei 85 °C während 1 1/2 Stunden. Die Analyse des Reaktionsgemisches zeigt, dass die Reaktion im wesentlichen beendet ist. Das Produkt wird als leicht gelbe Flüssigkeit durch Abdampfen der flüchtigen Anteile isoliert.

b) 2,72 g des unter (a) erhaltenen Produkts und 95 mg p-Toluolsulfonsäure werden unter Rühren einer Lösung von 2,6-Di-tert.-butyl-4-mercaptophenol in Toluol zugegeben und das Reaktionsgemisch bei 50-55 °C während 5 Stunden weitergerührt.

Die toluolische Lösung wird danach mit Wasser, gesättigter Natriumbicarbonat-Lösung und wieder mit Wasser gewaschen und über wasserfreiem Natriumsulfat getrocknet. Anschliessend wird das Trocknungsmittel abfiltriert und das Lösungsmittel bei 0,195 mbar abdestilliert. Das erhaltene flüssige Produkt wird in einem Lösungsmittelgemisch Heptan/Toluol (60 : 40) gelöst und chromatographisch gereinigt. Nach dem Abtrennen der flüchtigen Anteile bei 75-80 °C und 0,26 mbar (in ca. 4 Stunden) erhält man das Endprodukt als leicht gelbe Flüssigkeit.

Analyse für $C_{44}H_{74}O_2S_3$
Berechnet : C 72,27 H 10,20
Gefunden : C 72,47 H 10,37.

Beispiel 5 : 4-Methyl-2,2,4-tris-(3,5-di-tert.-butyl-4-hydroxyphenylthio)-pentan

a) 4-Methyl-4-(3,5-di-tert.-butyl-4-hydroxyphenylthio)-2-pentanon

8,48 g Mesityloxid (0,084 Mol) werden einer Lösung von 21,0 g 2,6-Di-tert.-Butyl-4-mercaptophenol (0,088 Mol) und 1 g Triethylamin in 30 ml Toluol zugetropft. Das Reaktionsgemisch wird auf 50-55 °C erwärmt und 6 Stunden reagieren gelassen. Das Lösungsmittel wird anschliessend unter Vakuum abgedampft und der Rückstand 2 Stunden bei 60-65 °C und 0,26 mbar stehen gelassen und danach in Toluol gelöst und chromatographisch (durch Silicagel) gereinigt. Das Produkt wird schliesslich durch Abdestillieren der flüchtigen Anteile im Vakuum bis zu einem Enddruck von 0,13 mbar nach 5 Stunden isoliert.

Analyse für $C_{20}H_{32}O_2S$
Berechnet : C 71,37 H 9,58
Gefunden : C 71,25 H 9,41.

b) 0,11 g Bor-trifluorid-etherat werden einer Lösung von 2,7 g des Produktes aus (a) und 4,14 g 2,6-Di-tert.-butyl-4-mercaptophenol in 30 ml Toluol zugegeben. Das Reaktionsgemisch wird 9 Stunden bei 55-65 °C und weitere 3 Stunden bei 105-110 °C reagieren gelassen. Die Toluolische Lösung wird mit Wasser gewaschen und über Natriumsulfat getrocknet. Der Rückstand wird durch Abdampfen des Lösungsmittels im Vakuum isoliert, in einem Lösungsmittelgemisch Heptan/Toluol (70 : 30) gelöst und chromatographisch (durch Silikagel) gereinigt. Das isolierte Produkt erstarrt beim Abkühlen und schmilzt bei 93-115 °C.

Analyse für $C_{48}H_{74}O_3S_3$
Berechnet : C 72,48 H 9,38
Gefunden : C 72,49 H 9,40.

Beispiel 6 : 1,1,3-Tris-(3-tert.-butyl-4-hydroxy-5-methylphenylthio)-butan

a) 3-(3-Tert.-butyl-4-hydroxy-5-methylphenylthio)-butan

Die Reaktion eines molaren Anteiles an n-Butyraldehyd mit einem molaren Anteil an 2-Tert.-butyl-6-methyl-4-mercaptophenol in Gegenwart von Triethylamin im wesentlichen gemäss der in Beispiel 3a beschriebenen Methode führt zu einem Produkt, welches dem gewünschten substituierten n-Butyraldehyd-Analogen des Produktes von Beispiel 3a entspricht.

b) Das obengenannte erfindungsgemässe Produkt erhält man in Analogie zum Produkt von Beispiel 3 durch Umsetzung eines molaren Anteiles am Produkt aus (a) mit zwei molaren Anteilen an 2-Tert.-butyl-6-methyl-4-mercaptophenol. Nach dem Umkristallisieren aus einem Lösungsmittelgemisch Heptan/Toluol 12 : 1 erhält man eine weisse kristalline Substanz mit Smp. 65-69 °C.
Analyse für $C_{37}H_{52}O_3S_3$
Berechnet : C 69,32 H 8,17 S 15,0
Gefunden : C 71,25 H 9,41 S 14,6.

Beispiel 7 : 1,1,3,5-Tetrakis-(3,5-di-tert.-butyl-4-hydroxyphenylthio)-hexan

a) 12,2 g (0,05 Mol) 2,6-Di-tert.-butyl-4-mercaptophenol, 2,4 g 2,4-Hexadienal (0,025 Mol) und 0,5 g Triethylamin werden zusammen bei 20 °C in 50 ml Toluol gelöst. Die erhaltene klare Lösung wird auf 55-65 °C erwärmt und 37 Stunden reagieren gelassen. Das Produkt wird durch Abdampfen der flüchtigen Anteile bei 0,26 mbar während einer Stunde isoliert. Die Analyse zeigt, dass es sich beim erhaltenen Produkt im wesentlichen um 3,5-Bis-(3,5-di-tert.-butyl-4-hydroxyphenylthio)-caproaldehyd handelt.

b) 0,17 g Bor-trifluorid-etherat werden einer Lösung aus 14,5 g des Produktes aus (a) und 12,2 g 2,6-Di-tert.-butyl-4-mercaptophenol unter Rühren bei Zimmertemperatur und danach bei 50-55 °C zugegeben. Das Reaktionsgemisch wird mit 50 ml Toluol verdünnt, zuerst mit Wasser, dann zweimal mit 2 N Natriumhydroxidlösung und erneut mit Wasser gewaschen und danach mit wasserfreiem Natriumsulfat getrocknet. Nach Entfernung des Trocknungsmittels und der flüchtigen Anteile wird das isolierte Produkt in einem heissen Lösungsmittelgemisch aus 70 ml n-Heptan und 20 ml Isopropanol gelöst und auskristallisieren gelassen. Man erhält eine weisse kristalline Substanz mit Smp. 160-162 °C.
Analyse für $C_{62}H_{94}O_4S_4$
Berechnet : C 72,18 H 9,18
Gefunden : C 72,3 H 9,3.

Beispiel 8 : 1,1,3-Tris-(3-methyl-5-tert.-butyl-4-hydroxyphenylthio)-cyclohexan

Das Verfahren von Beispiel 6 wird wiederholt, mit der einzigen Ausnahme, dass Crotonaldehyd durch die äquivalente Menge 2-Cyclohexen-1-on ersetzt wird. Man erhält die obengenannte erfindungsgemässe Verbindung.

Beispiel 9 : 1,1,3-Tris-(3,5-di-tert.-amyl-4-hydroxyphenylthio)-propan

Das Verfahren von Beispiel 3 wird wiederholt, mit der einzigen Ausnahme, dass Crotonaldehyd und 2,6-Di-tert.-butyl-4-mercaptophenol jeweils durch die äquivalente Menge Acrolein und 2,6-Di-tert.-amyl-4-mercaptophenol ersetzt werden. Man erhält die obengenannte erfindungsgemässe Verbindung.

Beispiel 10 : 1,1,3-Tris[3-tert.-butyl-5-(1,1,3,3-tetramethylbutyl)-4-hydroxyphenylthio]-3,5,5-trimethylcyclohexan

Das Verfahren von Beispiel 5 wird wiederholt, mit der einzigen Ausnahme, dass Mesityloxid und 2,6-Di-tert.-butyl-4-mercaptophenol jeweils durch die äquivalente Menge Isophoron und 2-tert.-Butyl-6-(1,1,3,3-tetramethylbutyl)-4-mercaptophenol ersetzt werden. Man erhält die obengenannte erfindungsgemässe Verbindung.

Beispiel 11 : 1,1,3-Tris-(3,5-di-tert.-butyl-4-hydroxyphenylthio)-3-phenylpropan

Das Verfahren von Beispiel 3 wird wiederholt, mit der einzigen Ausnahme, dass das Crotonaldehyd durch die äquivalente Menge Zimtaldehyd ersetzt wird. Man erhält die obengenannte erfindungsgemässe Verbindung.

Beispiel 12 : Stabilisierung von schlagfestem Polystyrol

Man stellt eine Lösung von 8 Gew.-% Polybutadien-Kautschuk (Firestone DIENE 55®) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121 °C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt :
eine Stunde bei 100 °C um die Anfangstemperatur vorzugeben, eine weitere Stunde, um 140 °C zu erreichen und anschliessend weitere 8 Stunden, wobei die Temperatur jede Stunde um 10 °C erhöht wird, bis schliesslich ein Maximum von 220 °C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200 °C für 45 Minuten bei einem Druck von 1,33 mbar belassen, um flüchtige Bestandteile zu entfernen. Nach dieser Behandlung wird der Block sofort in einer heizbaren hydraulischen Presse bei 205 °C zwischen zwei Aluminiumfolien zu einem dicken Stab gepresst (drei Minuten Erhitzen, fünf Minuten ungeheizt).

Der Stab wird mittels einer Bandsäge zerkleinert und anschliessend granuliert. Alle Polymermischungen werden bei 205 °C extrudiert und dann zerkleinert. Die Pellets werden unter Druck bei 205 °C in dehnbare 3,2 mm dicke Streifen verformt. Diese Probestreifen werden dann bei 150 °C in einem Umluftofen gealtert, wobei sie auf Glasplatten, die sich wiederum auf rotierenden Trägern befinden, aufgebracht sind. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts ; Ziehgeschwindigkeit : 5 mm/Minute, ASTM D-638).

In den folgenden Tabellen 1 und 2 sind die Ergebnisse der Dehnbarkeitsmessung und der Messungen des Yellowness-Index von bei 80 °C bzw. 150 °C ofengealterten Proben angegeben.

Tabelle 1 : Dehnbarkeitsmessungen und Yellowness Index von bei 80 °C ofengealterten Proben mit Stabilisator

| Additiv | Stabilisatormenge (Gew.-%) | Dehnbarkeit der Probe (%) und Dauer der Hitzebelastung (h) | | | | |
|---|---|---|---|---|---|---|
| | | 0 h | 300 h | 600 h | 900 h | 1200 h |
| keines | - | 33 | 9 | 3 | 3 | 3 |
| Produkt von Beispiel 1 | 0,1 | 42 | 36 | 23 | 12 | 10 |

| Additiv | Stabilisatormenge (Gew.-%) | Yellowness Index und Dauer der Hitzebehandlung (h) | | | | |
|---|---|---|---|---|---|---|
| | | 0 h | 300 h | 600 h | 900 h | 1200 h |
| keines | - | 7 | 14 | 45 | 59 | - |
| Produkt von Beispiel 1 | 0,1 | 11 | 22 | 36 | 43 | 45 |

Tabelle 2 : Dehnbarkeitsmessungen und Yellowness Index von bei 150 °C ofengealterten Proben mit Stabilisator

| Additiv (Produkt von Beispiel) | Stabilisatormenge (Gew.-%) | Dehnbarkeit der Probe (%) und Dauer der Hitzebelastung (h) | | | | |
|---|---|---|---|---|---|---|
| | | 0 h | 1/2 h | 1 h | 1 1/2 h | 2 h |
| keines | - | 33 | 7 | 7 | 3 | 3 |
| 1 | 0,1 | 42 | 36 | 30 | 22 | 10 |
| keines* | - | 25 | 13 | 4 | 3 | 3 |
| 3* | 0,1 | 65 | 60 | 52 | 42 | 33 |
| 4* | 0,1 | 72 | 47 | 29 | 18 | 7 |

| Additiv (Produkt von Beispiel) | Stabilisatormenge (Gew.-%) | Yellowness Index und Dauer der Hitzebehandlung (h) | | | | |
|---|---|---|---|---|---|---|
| | | 0 h | 1/2 h | 1 h | 1 1/2 h | 2 h |
| keines | - | 7 | 18 | 30 | 38 | 43 |
| 1 | 0,1 | 11 | 10 | 27 | 31 | 33 |
| keines * | - | -1,3 | -0,6 | 3,7 | 61 | - |
| 3* | 0,1 | 0 | 6 | 10 | 12 | 14 |
| 4* | 0,1 | -0,3 | 6 | 9 | 8 | 14 |

* Versuche zu einem späteren Zeitpunkt mit frischem Substratmaterial durchgeführt.

Beispiel 13 : Stabilisierung von Polypropylen (Lichtstabilität)

Unstabilisiertes Polypropylenpulver (Hercules Profax® 6501) wird mit 0,2 Gew.-% eines der in der nachfolgenden Tabelle 3 angegebenen Additive gemischt. Diese Mischung wird anschliessend bei 182 °C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220 °C und 12 bar zu einer 0,13 mm dicken Probe verformt. Diese Probe wird in einer Fluoreszenssonnenlicht/Schwarzlichtkammer bis zur Zersetzung belichtet. Als Zersetzungszeitpunkt wird diejenige Anzahl Stunden gewertet, bei der die IR-spektroskopisch gemessene dekadische Extinktion der Carbonylbande 50 % des Anfangswertes erreicht. Die Messdaten sind in der folgenden Tabelle 3 dargestellt.

Tabelle 3

| Additiv (Produkt von Beispiel) | Zahl der Stunden bis zur Zersetzung |
|---|---|
| keines | 200 - 300 |
| 1 | 530 |
| 2 | 450 |
| 3 | 480 |
| 4 | 470 |

Beispiel 14 : Stabilisierung von Polypropylen (Oxidationsstabilität)

Um die Oxidationsstabilität von Polypropylen, das 0,2 Gew.-% eines erfindungsgemässen Additivs oder einer synergistisch wirkenden Formulierung bestehend aus 0,1 Gew.-% eines Additivs und 0,3 Gew.-% Distearylthiodipropionat (DSTDP) enthält, zu untersuchen, werden Plättchen von 0,64 mm Dicke hergestellt und in einem Umluftofen bei 150 °C gealtert. Als Zersetzungszeitpunkt wird diejenige Anzahl von Stunden gewertet, nach der die Probe die ersten Zerfallserscheinungen zeigt (braune Ecken, Risse). Die Messdaten sind in Tabelle 4 dargestellt :

Tabelle 4

| Additiv (Produkt von Beispiel) | Zahl der Stunden bis zur Zersetzung der Proben | |
|---|---|---|
| | 0,2 % Additiv | 0,1 % Additiv + 0,3 % DSTDP |
| 1 | 120 | 250 |
| 2 | 220 | 370 |
| 3 | 260 | 490 |
| 4 | 360 | 540 |
| keines | 3 | |
| 0,3 % DSTDP | 60 - 90 | |

Beispiel 15 : Stabilisierung von Schmiermitteln

a) Es wird der Oel-Oxidationstest, Standard Version nach ASTM D 2272 (Rotary Bomb Oxidation Test), in folgender Weise durchgeführt. Eine Oelprobe von 50 ml Mineralöl Exxon 1243® wird unter Zusatz von 0,25 g eines der in der nachfolgenden Tabelle 5 angegebenen Additive in einem Glasgefäss zusammen mit 5 ml destilliertem Wasser und einer blankpolierten, mit Petrolether gewaschenen katalytisch wirkenden Cu-Spirale in einer Sauerstoff-Atmosphäre oxydiert.

Das Glasgefäss befindet sich in einer Bombe aus rostfreiem Stahl mit Manometer. Die Bombe dreht sich axial mit 100 U/Min. in einem Winkel von 30 °C zur Horizontalen, in einem Oelbad bei 150 °C. Der Sauerstoffdruck beträgt anfangs, vor dem Aufheizen ca. 6 bar, steigt bei 150 °C auf knapp 14 bar und bleibt bis zur einsetzenden Oxidation konstant. Die Prüfung ist beendet bei einem Druckabfall um 1,7 bar. Aufgezeichnet wird die Zeit in Minuten. Die Ergebnisse sind in Tabelle 5 wiedergegeben :

Tabelle 5

| Additiv (Produkt von Beispiel) | Minuten bis Druckabfall um 1,7 bar |
|---|---|
| keines | 31 |
| 3 | 166 |
| 4 | 201 |
| 7 | 177 |

b) Turbinenöl-Oxidationstest : Eine Oelprobe von 300 ml Turbinenöl Exxon LO 5084® enthaltend 0,25 Gew.-% der Verbindung von Beispiel 4 und 60 ml destilliertes Wasser wird im Oel-Oxidationstest

gemäss ASTM D 943-81 geprüft. Bestimmt wird die Zeit (in Stunden) bis zur Zersetzung der Oelprobe, d. h. bis zur Erreichung einer Säurezahl von 2,0. Die Resultate sind in der nachstehenden Tabelle 6 aufgeführt :

Tabelle 6

| Additiv (Produkt von Beispiel) | Stunden bis zur Erreichung von Säurezahl 2,0 |
|---|---|
| keines | 130 |
| 4 | 1800 |

**Patentansprüche**

1. Verbindungen der Formel I

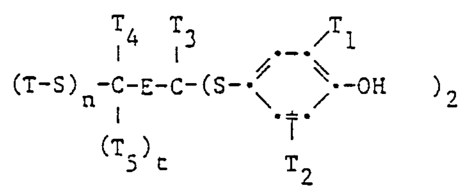

(I)

worin

$T_1$ und $T_2$ unabhängig voneinander $C_1$-$C_{12}$-Alkyl, $C_5$-$C_6$-Cycloalkyl, unsubstituiertes oder durch $C_1$-$C_{12}$-Alkyl substituiertes Phenyl, unsubstituiertes oder durch $C_1$-$C_{12}$-Alkyl substituiertes $C_7$-$C_9$-Aralkyl bedeuten und $T_2$ zusätzlich Wasserstoff sein kann,

T $C_1$-$C_{24}$-Alkyl, unsubstituiertes oder durch $C_1$-$C_{12}$-Alkyl substituiertes Phenyl oder eine Gruppe

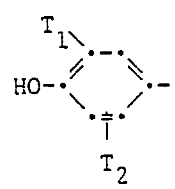

ist,

E eine direkte Bindung, $C_1$-$C_{11}$-Alkylen, $C_5$-$C_6$-Cycloalkylen, Phenylen oder die Gruppe

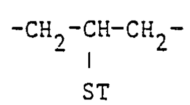

bedeutet,

$T_3$ und $T_4$ unabhängig voneinander Wasserstoff, $C_1$-$C_{30}$-Alkyl, welches unsubstituiert oder durch unsubstituiertes oder durch $C_1$-$C_{12}$-Alkyl substituiertes Phenyl substituiert sein kann, oder $C_5$-$C_6$-Cycloalkyl sind oder, wenn E Methylen bedeutet, $T_3$ und $T_4$ zusammen geradkettiges oder verzweigtes $C_3$-$C_7$-Alkylen bilden,

$T_5$ Wasserstoff, $C_1$-$C_4$-Alkyl oder Phenyl ist,

n die Zahlen 1 oder 2 und

t Null oder 1 sind, wobei $t = 2 - n$ sein muss.

2. Verbindungen gemäss Anspruch 1, der Formel I, worin $T_2$ in ortho-Stellung zur Hydroxygruppe steht.

3. Verbindungen gemäss Anspruch 2, der Formel I, worin $T_1$ und $T_2$ unabhängig voneinander $C_1$-$C_8$-Alkyl bedeuten.

4. Verbindungen gemäss Anspruch 2, der Formel I, worin $T_1$ Methyl oder tert.-Butyl und $T_2$ tert.-Butyl bedeuten.

5. Verbindungen gemäss Anspruch 4, der Formel I, worin $T_1$ tert.-Butyl bedeutet.

6. Verbindungen gemäss Anspruch 1, worin T $C_7$-$C_{18}$-Alkyl oder eine Gruppe

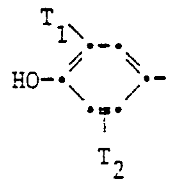

bedeutet, worin $T_1$ und $T_2$ die in Anspruch 1 angegebene Bedeutung haben.

7. Verbindungen gemäss Anspruch 1, der Formel I, worin E eine direkte Bindung, $C_1$-$C_6$-Alkylen oder die Gruppe

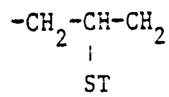

ist, worin T die in Anspruch 1 angegebene Bedeutung hat.

8. Verbindungen gemäss Anspruch 1, der Formel I, worin $T_3$ und $T_4$ unabhängig voneinander Wasserstoff oder $C_1$-$C_4$-Alkyl sind oder, wenn E Methylen ist, $T_3$ und $T_4$ zusammen Trimethylen oder 2,2-Dimethylpropan-1,3-diyl bilden.

9. Verbindungen gemäss Anspruch 1, der Formel I, worin $T_5$ Wasserstoff oder Methyl bedeutet.

10. Verbindungen gemäss Anspruch 1 mit folgender Bezeichnung
1,1,2,2-Tetrakis-(3,5-di-tert.-butyl-4-hydroxyphenylthio)-ethan,
1,1,5,5-Tetrakis-(3,5-di-tert.-butyl-4-hydroxyphenylthio)-pentan,
1,1,3-Tris-(3,5-di-tert.-butyl-4-hydroxyphenylthio)-butan,
1,1-Bis-(3,5-di-tert.-butyl-4-hydroxyphenylthio)-3-(n-dodecylthio)-butan
1,1,3-Tris-[3-tert.-butyl-5-(1,1,3,3-tetramethylbutyl)-4-hydroxyphenylthio]-3,5,5-trimethylcyclohexan.

11. Stoffzusammensetzung enthaltend ein gegen oxidativen, thermischen oder strahlungsinduzierten Abbau empfindliches organisches Material und mindestens eine Verbindung der Formel I gemäss Anspruch 1 als Stabilisator.

12. Stoffzusammensetzung gemäss Anspruch 11, worin unter dem organischen Material ein Polymer zu verstehen ist.

13. Stoffzusammensetzung gemäss Anspruch 12, worin unter dem Polymer ein homopolymeres oder copolymeres Polyolefin zu verstehen ist.

14. Stoffzusammensetzung gemäss Anspruch 12, worin unter dem Polymer Polystyrol, Acrylnitril/Butadien/Styrol, Styrol/Butadien-Kautschuk, natürlicher Kautschuk, Polyisopren oder Polyester zu verstehen ist.

15. Stoffzusammensetzung gemäss Anspruch 11, worin unter dem organischen Material ein Mineralöl oder ein synthetisches Oel zu verstehen ist.

16. Stoffzusammensetzung gemäss Anspruch 11, enthaltend zusätzlich mindestens einen Costabilisator ausgewählt aus der Gruppe bestehend aus Antioxidantien, Lichtschutzmittel, Metalldesaktivatoren, Phosphite, Phosphonite und Thiosynergisten.

**Claims**

1. A compound of the formula I

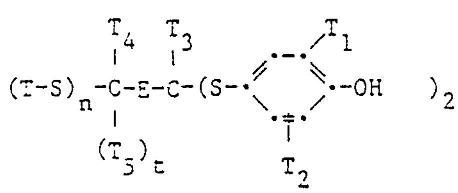

(I)

wherein

$T_1$ and $T_2$ independently of one another are $C_1$-$C_{12}$alkyl, $C_5$-$C_6$cycloalkyl, unsubstituted or $C_1$-$C_{12}$alkyl-substituted phenyl, unsubstituted or $C_1$-$C_{12}$alkyl-substituted $C_7$-$C_9$aralkyl and $T_2$ may additionally be hydrogen, T is $C_1$-$C_{24}$alkyl, unsubstituted or $C_1$-$C_{12}$-alkyl-substituted phenyl or a group

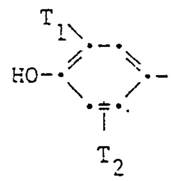

E is a direct bond, $C_1$-$C_{11}$alkylene, $C_5$-$C_6$cycloalkylene, phenylene or the group

$-CH_2-CH-CH_2-$
|
ST

$T_3$ and $T_4$ independently of one another are hydrogen, $C_1$-$C_{30}$alkyl which may be unsubstituted or substituted by unsubstituted or $C_1$-$C_{12}$-alkyl-substituted phenyl, or $C_5$-$C_6$cycloalkyl or, when E is methylene, $T_3$ and $T_4$ together form straight chain or branched $C_3$-$C_7$alkylene,

$T_5$ is hydrogen, $C_1$-$C_4$alkyl or phenyl,

n is the numbers 1 or 2 and

T is zero or 1, where t must be 2—n.

2. A compound according to Claim 1, of the formula I wherein $T_2$ is in the ortho position to the hydroxyl group.

3. A compound according to Claim 2, of the formula I wherein $T_1$ and $T_2$ independently of one another are $C_1$-$C_8$alkyl.

4. A compound according to Claim 2, of the formula I wherein $T_1$ is methyl or tert-butyl and $T_2$ is tert-butyl.

5. A compound according to Claim 4, of the formula I wherein $T_1$ is tert-butyl.

6. A compound according to Claim 1, wherein T is $C_7$-$C_{18}$alkyl or a group

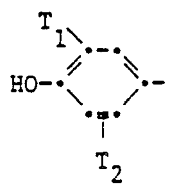

wherein $T_1$ and $T_2$ are as defined in Claim 1.

7. A compound according to Claim 1, of the formula I wherein E is a direct bond, $C_1$-$C_6$alkylene or the group

$-CH_2-CH-CH_2$
|
ST

wherein T is as defined in Claim 1.

8. A compound according to Claim 1, of the formula I wherein $T_3$ and $T_4$ independently of one another are hydrogen or $C_1$-$C_4$alkyl or, when E is methylene, $T_3$ and $T_4$ together form trimethylene or 2,2-dimethyl propan-1,3-diyl.

9. A compound according to Claim 1, of the formula I wherein $T_5$ is hydrogen or methyl.

10. A compound according to Claim 1 having the following designation

1,1,2,2-tetrakis-(3,5-di-tert-butyl-4-hydroxyphenylthio)-ethane,

1,1,5,5-tetrakis-(3,5-di-tert-butyl-4-hydroxyphenylthio)-pentane,

1,1,3-tris-(3,5-di-tert-butyl-4-hydroxyphenylthio)-butane,

1,1-bis-(3,5-di-tert-butyl-4-hydroxyphenylthio)-3-(n-dodecylthio)-butane or

1,1,3-tris-[3-tert-butyl-5-(1,1,3,3-tetramethylbutyl)-4-hydroxyphenylthio]-3,5,5-trimethylcyclohexane.

11. A composition of matter containing an organic material which is sensitive to oxidative, thermal or radiation-induced degradation and at least one compound of the formula I according to Claim 1 as stabilizer.

12. A composition of matter according to Claim 11, wherein the organic material is to be understood as meaning a polymer.

13. A composition of matter according to Claim 12, wherein the polymer is to be understood as meaning a homopolymeric or copolymeric polyolefin.

14. A composition of matter according to Claim 12, wherein the polymer is to be understood as meaning polystyrene, acrylonitrile/butadiene/styrene, styrene/butadiene rubber, natural rubber, polyisoprene or polyester.

15. A composition of matter according to Claim 11, wherein the organic material is to be understood as meaning a mineral oil or a synthetic oil.

16. A composition of matter according to Claim 11, containing in addition at least one costabilizer selected from the group consisting of antioxidants, light stabilizers, metal deactivators, phosphites, phosphonites and thiosynergists.

## Revendications

1. Composés répondant à la formule I :

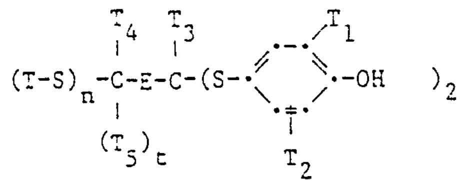

(I)

dans laquelle

$T_1$ et $T_2$ représentent chacun, indépendamment l'un de l'autre, un alkyle en $C_1$-$C_{12}$, un cyclo-alkyle en $C_5$ ou $C_6$, un phényle non substitué ou porteur d'un alkyle en $C_1$-$C_{12}$, ou un aralkyle en $C_7$-$C_9$ non substitué ou porteur d'un alkyle en $C_1$-$C_{12}$, et $T_2$ peut en outre représenter l'hydrogène,

T représente un alkyle en $C_1$-$C_{24}$, un phényle non substitué ou porteur d'un alkyle en $C_1$-$C_{12}$, ou un radical de formule :

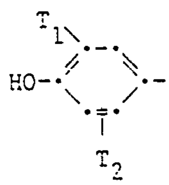

E représente une liaison directe, un alkylène en $C_1$-$C_{11}$, un cycloalkylène en $C_5$ ou $C_6$, un phénylène ou un radical de formule :

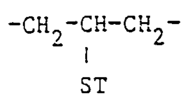

$T_3$ et $T_4$ représentent chacun, indépendamment l'un de l'autre, l'hydrogène, un alkyle en $C_1$-$C_{30}$, non substitué ou porteur d'un phényle, lui-même non substitué ou porteur d'un alkyle en $C_1$-$C_{12}$, ou un cycloalkyle en $C_5$ ou $C_6$, ou encore, lorsque E représente un radical méthylène, $T_3$ et $T_4$ peuvent former ensemble un alkylène en $C_3$-$C_7$ linéaire ou ramifié,

$T_5$ représente l'hydrogène, un alkyle en $C_1$-$C_4$ ou un phényle,

n représente le nombre 1 ou le nombre 2 et

t est égal à 0 ou à 1, avec la condition que t et n satisfassent à la relation $t = 2 - n$.

2. Composés de formule I selon la revendication 1 dans lesquels $T_2$ se trouve en position ortho par rapport au radical hydroxy.

3. Composés de formule I selon la revendication 2 dans lesquels $T_1$ et $T_2$ représentent chacun, indépendamment l'un de l'autre, un radical alkyle en $C_1$-$C_8$.

4. Composés de formule I selon la revendication 2 dans lesquels $T_1$ représente un radical méthyle ou tert-butyle et $T_2$ un radical tert-butyle.

5. Composés de formule I selon la revendication 4 dans lesquels $T_1$ représente un radical tert-butyle.

6. Composés selon la revendication 1 dans lesquels T représente un alkyle en $C_7$-$C_{18}$ ou un radical de formule :

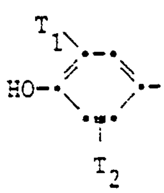

dans lequel $T_1$ et $T_2$ ont les significations qui leur ont été données à la revendication 1.

7. Composés de formule I selon la revendication 1 dans lesquels E représente une liaison directe, un alkylène en $C_1$-$C_6$ ou un radical de formule :

$$-CH_2-\underset{\substack{| \\ ST}}{CH}-CH_2-$$

dans lequel T a la signification qui lui a été donnée à la revendication 1.

8. Composés de formule I selon la revendication 1 dans lesquels $T_3$ et $T_4$ représentent chacun, indépendamment l'un de l'autre, l'hydrogène ou un alkyle en $C_1$-$C_4$, ou encore, lorsque E désigne un radical méthylène, $T_3$ et $T_4$ forment ensemble un radical triméthylène ou diméthyl-2,2 propane-diyle-1,3.

9. Composés de formule I selon la revendication 1 dans lesquels $T_5$ représente l'hydrogène ou un méthyle.

10. Composés selon la revendication 1 qui ont les noms suivants :
Tétrakis-(di-tert-butyl-3,5 hydroxy-4 phénylthio)-1,1,2,2 éthane,
Tétrakis-(di-tert-butyl-3,5 hydroxy-4 phénylthio)-1,1,5,5 pentane,
Tris-(di-tert-butyl-3,5 hydroxy-4 phénylthio)-1,1,3 butane,
Bis-(di-tert-butyl-3,5 hydroxy-4 phénylthio)-1,1 n-dodécylthio-3 butane et
Tris-[tert-butyl-3 (tétraméthyl-1,1,3,3 butyl)-5 hydroxy-4 phénylthio]-1,1,3 triméthyl-3,5,5 cyclohexane.

11. Composition qui contient une matière organique susceptible d'être dégradée sous l'action de l'oxydation, de la chaleur ou d'un rayonnement, et, comme stabilisant, au moins un composé de formule I selon la revendication 1.

12. Composition selon la revendication 11 dans laquelle la matière organique est un polymère.

13. Composition selon la revendication 12 dans laquelle le polymère est une polyoléfine homopolymère ou copolymère.

14. Composition selon la revendication 12 dans laquelle le polymère est le polystyrène, un terpolymère acrylonitrile/butadiène/styrène, un caoutchouc styrène/butadiène, un caoutchouc naturel, le poly-isoprène ou un polyester.

15. Composition selon la revendication 11 dans laquelle la matière organique est une huile minérale ou une huile synthétique.

16. Composition selon la revendication 11 qui contient, en plus, ou moins un costabilisant pris dans l'ensemble constitué par les anti-oxydants, les stabilisants à la lumière, les désactivants de métaux, les phosphites, les phosphonites et les agents de synergie sulfurés.